(19) Europäisches Patentamt
European Patent Office
Office européen des brevets

(11) Publication number: **0 019 260**
**A1**

(12) # EUROPEAN PATENT APPLICATION

(21) Application number: 80102633.7

(51) Int. Cl.³: C 07 D 213/80

(22) Date of filing: 12.05.80

(30) Priority: 18.05.79 IT 2279779

(43) Date of publication of application: 26.11.80 Bulletin 80/24

(84) Designated Contracting States: AT CH DE GB LI NL SE

(71) Applicant: Laboratorio Guidotti & C. S.p.A.
Via Trieste 40
I-50100 Pisa(IT)

(72) Inventor: Cerbai, Guido
Via Brodolini 10
Pisa(IT)

(72) Inventor: Sagramora, Giorgio
Via Parini 6
Pisa(IT)

(74) Representative: Bühling, Gerhard, Dipl.-Chem. et al,
Patentanwaltsbüro Tiedtke-Bühling-Kinne
Grupe-Pellmann Bavariaring 4
D-8000 München 2(DE)

(54) **A process for the preparation of D-glucitol hexanicotinate.**

(57) A process for the preparation of D-glucitol hexanicotinate having the formula:

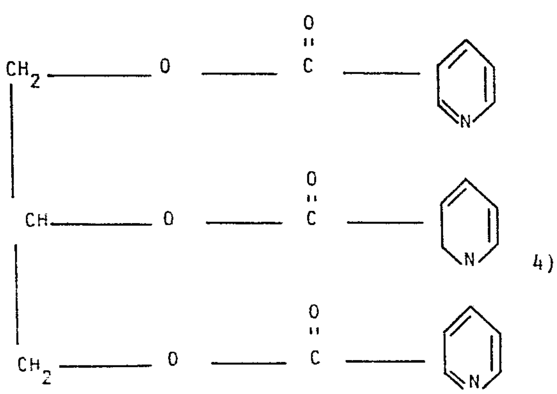

(1)

wherein nicotinic acid is reacted with D-glucitol, using nicotinamide as the catalist, in the presence of $POCl_3$, and in py-ridine, the process being improved both as regards the reaction pattern and as regards the yield by having recourse to specific rea-ction conditions, particularly as regards time, temperature and molar ratio of reactans.

EP 0 019 260 A1

"A process for the preparation of D-glucitol hexanicotinate"

* * * * * *

The present invention relates to a process for the preparation of D-glucitol hexanicotinate, having the formula:

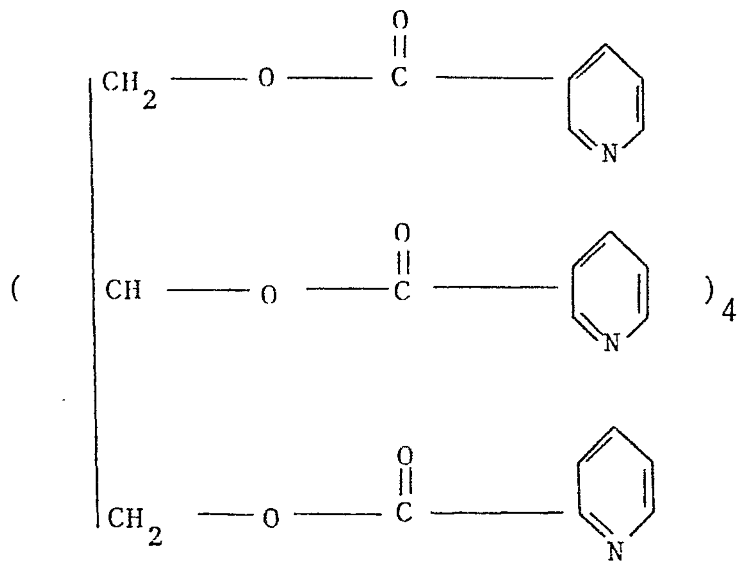

(1)

This compound has been previously studied by the Applicant and is known in the pharmacological and therapeutical fields as hypocholesteremic and thus useful for the treatment of artheriosclerosis diseases.

To date the industrial preparation of this compound takes place in two steps, as resumed in the following scheme:

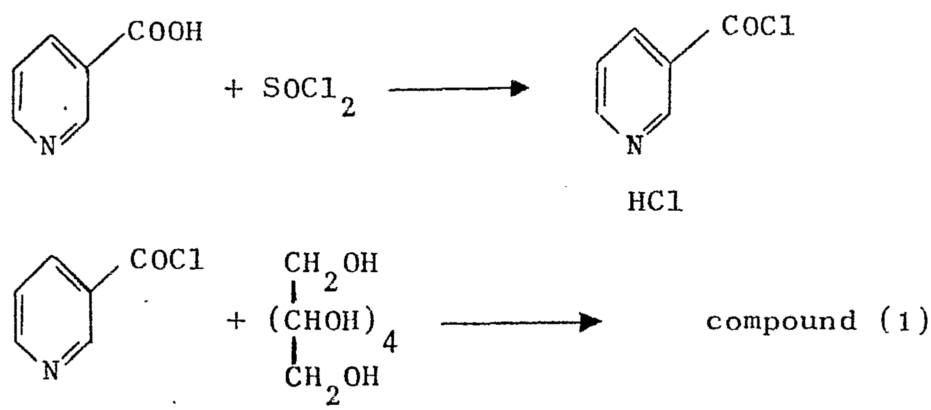


BAD ORIGINAL

Thus, according to this method, the steps are provided of chlorinating nicotinic acid, recovering and purifying the chloride and then reacting it with D-glucitol, the yields (as referred to the nicotinic acid) being 73-74%.

This industrial process, however, raises several problems and drawbacks, as hereinafter shortly resumed.

1) The chlorination of the nicotinic acid is carried out with a relevant excess of $SO\,Cl_2$ and requires, for the completion of the chlorination, an extended heating to reflux (about 18 hours).

Of course the excess of $SO\,Cl_2$ has consequences as regards the polluting charge of the process effluents.

Furthermore, (as confirmed by the literature: see for example Heterocyclic Compounds, Pyridine and Derivative, Part I, pag. 45; Part III pag. 210 ), the long time needed for the reaction between nicotinic acid and $SO\,Cl_2$, causes a partial chlorination of the pyridine ring and gives place up to 60% of a mixture of 5-chloro- and 5,6-dichloro-pyridinic acids.

The main consequence is that more or less remarkable traces of chlorinated products are present in the final D-glucitol hexanicotinate, which can be difficultly removed by the common purification methods.

2) The isolation and purification step of the final chloride hydrochloride involves the filtration of a product fuming in air (HCl vapours), with the attendant drawbacks of environmental pollution and of danger for the people attending to the plant.

3) In the esterification phase the resulting product may be in resinous form (owing to the presence of impurities of several types, particularly of partial esters), thus making it necessary a further purification step to obtain a crystalline product, suitable for the therapeutical use.

BAD ORIGINAL

According to the prior art, besides the U.K. patent specification No. 932,079 of the same Applicant, the Swiss patent 371,450 and the French Patent No. 1,481,007, which disclose the preparation of D-glucitol hexanicotinate by reacting D-glucitol and nicotinoyl chloride hydrochloride as prepared through the conventional manner, the preparation of nicotinic esters by direct esterification of the acid with a polyalcohol is described in the Japanese patent pubblication No. 2359/67 of February 2, 1967.

In this case, there is mainly described and claimed the direct esterification of nicotinic acid with pentaerythritol, but among the like esterification reactions also that with D-glucitol is included.

The specific feature of the method claimed in this patent is that the esterification is carried out by directly starting from the acid and the polyalcohol, in the presence of p-toluen-sulfonychloride, with an extended heating time.

Among the esterification reactions directly involving the nicotinic acid, the following can be cited:

(a) U.K patent specification No. 1,053,689. There is described the reaction between nicotinic acid and inositol in pyridine in the presence of $POCl_3$, but without the intervention of catalysts. No description is given as regards the application of this method to the D-glucitol.

According to an experimental test, this method in the case of D-glucitol hexanicotinate gives place to yields which are considerably lower (about 25% less) than those of the inositol hexanicotinate.

(b) Japanese patent No. 7,006,017 of February 28,1970. There is described the preparation of inositol hexanicotinate starting from nicotinic acid and inositol,in pyridine and in the presence of $POCl_3$,nicotinamide being used as the catalyst; the like reaction with D-glucitol is not de-

BAD ORIGINAL

scribed.

If this method is used for the reaction of nicotinic acid with D-glucitol under the same conditions and with the same molar rations between the reacting substances, as indicated in the patent, there have been obtained yields not higher than 67%, referred to the D-glucitol, whereby the use of this method in the case of D-glucitol is unsuitable.

It has been now found and is the subject of the present invention a pro-cess for the preparation of D-glucitol hexanicotinate, wherein nicotinic acid and sorbitol are reacted in pyridine in the presence of $POCl_3$, nicotinamide being used as the catalyst, characterized in that the molar ratio between nicotinic acid and D-glucitol is at least 7.4 : 1; that the reaction mixture is heated for a time of between 230 and 280 minutes to a temperature of 78-85°C; that the reaction mixture of nicotinic acid and $POCl_3$, before the addition of D-glucitol, is diluted with pyridine and the reaction product is isolated by pouring, as small portions and under vigorous stirring, the reaction mixture in cold water and by filtering under vaquum.

As it will appear more detailedly from the following example, among the advantages of the process of the present invention, the following can be cited:

- the recovering and purifying steps in the preparation of the chloride of the nicotinic acid are eliminated, thus permitting a relevant reduction of the production costs, since there is not only reduced the reaction time and consequently the reaction cost, but also the pollution degree;
- there is obtained a final, product having a relevantly higher purity as regards the partial esters of D-glucitol (particularly tetra- and penta-esters);
- there are avoided impurities consisting of hexanicotinate partially chlorinated at the pyridine ring.

BAD ORIGINAL

As already mentioned, the results and advantages of the present invention are the direct consequence of the particular and wholly unforeseable conditions under which the esterification reaction is carried out according to the present invention. More particularly these conditions comprise a reaction time of 4 hours and a heating of the reaction mixture to a temperature of 80°C.

The unforeseability of the results of the present invention is confirmed by the comparison data with respect to the process of the Japanese patent No. 7,006,017.

In fact, if the esterification reaction is carried out by heating the reaction mixture to 60°C for 2 hours and to 80°C for 1 hour, the yield of ester, referred to the D-glucitol, is only 67%.

According to the preferred embodiment of the process of the present invention, the specific conditions under which the esterification reaction takes place are as follows:

- Heating temperature of reaction mixture: 80°C.
- Heating tme: 240 minutes.
- Dilution with pyridine of the reaction mixture: about one third by weight of the reaction mixture, before the addition of the D-glucitol.

Example

A four neck, 500 ml flask, having a cooling system, a mechanical stirrer, a thermometer and a drop feeding device, is charged with 40.0 g (0.326 moles) of nicotinic acid, 0.8 g of nicotinamide and 80.0 ml of pyridine, and the suspension is stirred at room temperature.

The internal temperature of the flask is brought to 40-42°C, by means of a heating bath at 50°C, and there is started the dropwise addition of 27.40 g of phosphorus oxychloride,(16.4 ml corresponding to 0.0179 moles). The dropwise addition is effected at a rate such as the internal temperature of the

BAD ORIGINAL

reaction mixture, which is maintained under stirring, is of 45-50°C, whereby the addition is effected in a time of 30-35 minutes.

The resulting reaction mixture, of yellow-pink colour, which is dense but can be stirred, id dropwise supplemented with 50 ml of pyridine, the internal temperature being controlled at a value of about 50°C.

At the end of this addition, the reaction mixture is supplemented with 8g of D-glucitol (0.44 moles), the addition taking place in small portions and at a rate such as the internal temperature is maintained at 55-60°C.

This addition is completed within 25-30 minutes.

After the reaction mixture has been stirred for one hour,the internal temperature of the mixture is raised to 80-82°C and the stirring is continued for further 4 hours, starting from the time at which the temperature of the reaction mixture has reached the steady value of 80 to 82°C.

After this time, the reaction mixture, which is now dense and of red-brown colour, although still stirrable, is cooled down to room temperature and then poured, in small portions and under vigorous stirring, in 500 mls of cold water; as the pouring is continued,a precipitate is separated which, at the end of the pouring step (involving about 25-30 minutes), is filtered under reduced pressure.

The residue is a solid white substance, which is washed with a double volume of water, filtered again and well squeezed on the filter.

The resulting solid, after a pre-drying under vacuum for a night in the presence of $CaCl_2$, is dehydrated until a constant weight is obtained, under vacuum and at 80-90°C.

There are obtained 27.55 g of D-glucitol hexanicotinate, having melting point of 212-216°C; the yield, as referred to the D-glucitol, is 77.11%.

BAD ORIGINAL

C L A I M S

1. A process for the preparation of D- glucitol hexanicotinate having the formula:

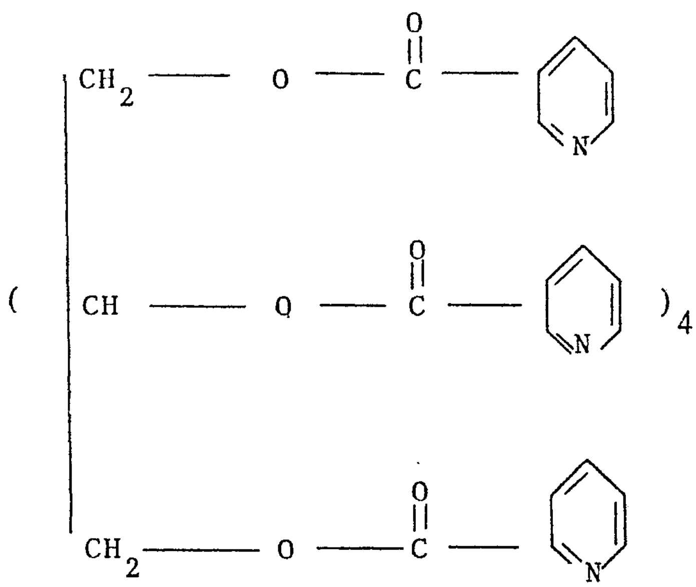

(1)

wherein, by using nicotinamide as the catalyst, D-glucitol is directly reacted with the reaction mixture resulting from nicotinic acid and $POCl_3$ in the presence of pyridine, characterized in that the molecular ration between nicoti ni-c acid and D-glucitol is at least 7.4 : 1; that the rea ction mixture is diluted with pyridine before the addition of D-glucitol; that the rea-ction mixture after the addition of D-glucitol is heated for a time of between 230 and 280 minutes to a temperature of 78-85°C, and that the reaction produ-ct is isolated by pouring, in small fractions and under vigorous stirring, the final rea-ction mixture in cold water and filtering the resulting suspension.

2. A process according to claim 1, characterized in that said heating time is 240 minutes and the said temperature is 80-82°C.

3. A process according to claim 1, characterized in that said dilution with pyridine is carried out by adding an amount of pyridine corresponding to one third by weight of the rea-ction mixture before the addition of D-glucitol.

4. A process according to claim 3, chara-cterized in that the addition of pyridine is carried out by controlling the temperature of the rea-ction mixture at a value of about 50°C.

European Patent Office

# EUROPEAN SEARCH REPORT

0019260

Application number

EP 80 10 2633

| Category | DOCUMENTS CONSIDERED TO BE RELEVANT: Citation of document with indication, where appropriate, of relevant passages | Relevant to claim | CLASSIFICATION OF THE APPLICATION (Int. Cl. 3) |
|---|---|---|---|
| | DE - A - 1 921 525 (W. HERBRAND et al.)<br>* Claim 5 *<br>-- | 1 | C 07 D 213/80 |
| | BE - A - 697 967 (STERWIN A.G.)<br>* Pages 10-11 *<br>-- | 1 | |
| | FR - M - 6014 (E.R.P.H.A.R.)<br>* Page 1 *<br>-- | 1 | TECHNICAL FIELDS SEARCHED (Int.Cl. 3) |
| D | JAPANESE PATENT DERWENT 2-2-1967 vol. 6, no. 5<br>& JP - B - 2359/67 (YOSHITOMI PHARM. CO LTD)<br>-- | 1 | C 07 D 213/80 |
| A | JAPANESE PATENT REPORT DERWENT vol. R10<br>& JP - B - 70 06017 (TOKYO TANABE CO. LTD)<br>-- | 1 | |
| D | US - A - 3 557 130 (B.G. PETTERSON)<br>* Columns 1-2 *<br>-- | 1 | CATEGORY OF CITED DOCUMENTS |
| D | CH - A - 371 450 (EPROVA AK)<br>* Example 1 *<br>---- | 1 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

✓ The present search report has been drawn up for all claims

| Place of search | Date of completion of the search | Examiner |
|---|---|---|
| The Hague | 08-08-1980 | BRIGHENTI |